Europäisches Patentamt

European Patent Office

Office européen des brevets

(19)

(11) Veröffentlichungsnummer: **0 159 410 B1**

(12) **EUROPÄISCHE PATENTSCHRIFT**

(45) Veröffentlichungstag der Patentschrift: **19.09.90**

(51) Int. Cl.⁵: **A 61 L 27/00** // A61F2/32

(21) Anmeldenummer: **84116032.8**

(22) Anmeldetag: **21.12.84**

(54) **Gelenkimplantat.**

(30) Priorität: **27.03.84 CH 1533/84**

(43) Veröffentlichungstag der Anmeldung:
**30.10.85 Patentblatt 85/44**

(45) Bekanntmachung des Hinweises auf die
Patenterteilung:
**19.09.90 Patentblatt 90/38**

(84) Benannte Vertragsstaaten:
**AT BE DE FR GB IT NL SE**

(56) Entgegenhaltungen:
**DE-A-2 811 603**
**FR-A-1 471 000**
**GB-A-2 025 238**

**CHEMICAL ABSTRACTS, Band 100, Nr. 5, 1.
Januar 1984, Seite 310, Nr. 39562t, Columbus,
Ohio, US; Y.W.S. NIE et al.: "Effects of titanium
niride, titanium and cobalt artificial joint
material on cells cultured in vitro" & BAICHIUEN
YIKE DAXUE XUEBAO 1982, 8(5), 21-4
IDEM**

(73) Patentinhaber: **GEBRÜDER SULZER
AKTIENGESELLSCHAFT
Zürcherstrasse 9
CH-8401 Winterthur (CH)**

(72) Erfinder: **Hintermann, Hans Erich, Dr.
Fluhweg 18
CH-3232 Ins (CH)**
Erfinder: **Semlitsch, Manfred, Dr.
Endlikerstrasse 86
CH-8400 Winterthur (CH)**

(74) Vertreter: **Sparing Röhl Henseler Patentanwälte
European Patent Attorneys
Rethelstrasse 123
D-4000 Düsseldorf 1 (DE)**

Courier Press, Leamington Spa, England.

## Beschreibung

Die Erfindung betrifft ein Gelenkimplantat für die Implantation in den lebenden Körper.

In dem Referat Nr. 39562t aus "Chemical Abstracts", Band 100, Nr. 5, 1. Januar 1984, Seite 310, wird aufgrund der biologischen Wirkungen von Titannitrid (TiN) im Vergleich zu denjenigen von metallischem Kobalt (Co) oder Titan (Ti) die Verwendung von Titannitrid für Gelenkendoprothesen empfohlen. Untersuchungen der Anmelderin haben jedoch gezeigt, dass Verhalten und Eigenschaften — beispielsweise bezüglich ihrer Gleiteigenschaften und/oder, bei Beschichtungen, bezüglich der Haftung auf einem Substrat — von gegeneinander laufenden Gleitflächen aus Titannitrid nicht optimal sind.

In der FR-A-1.471.000 werden Implantate und Prothesen beschrieben, die aus keramischen Werkstoffen — unter anderem auch aus Titankarbid und Titannitrid — bestehen sollen, weil keramische Werkstoffe sich als bioinert und biokompatibel erwiesen haben. Ueber das Reibungsverhalten zweier gegeneinander laufender Gleitpartner aus diesen keramischen Werkstoffen werden keine Angaben gemacht.

Bei hartstoffbeschichteten Gleitflächen von Gelenkimplantaten hat die Praxis nun gezeigt, dass Abrieb nicht vollständig vermieden werden kann; dabei können sich Schwierigkeiten beim Ersetzen eines Implantatteiles mit einer unter Umständen infolge Verschleiss nicht mehr voll wirksamen Schutzschicht ergeben. Aufgabe der Erfindung ist es, diese Schwierigkeiten möglichst weitgehend zu eliminieren. Die Erfindung besteht daher aus zwei Gleitpartnern mit aufeinander gleitend bewegten Gleitflächen, die mit einer unterschiedlichen Hartstoffbeschichtung aus einem Metallkarbid, -nitrid, -carbonitrid, -borid oder -silizid versehen sind, wobei der weichere der beiden Hartstoffe auf den operativ leichter auswechselbaren Gleitpartner aufgebracht ist.

Da Abrieb vorwiegend bei einem weicheren Gleitpartner auftritt, wird mit der Erfindung erzwungen, dass Verschleiss vor allem an demjenigen Implantatteil auftritt, der operativ leichter ersetzbar und daher mit geringerem Aufwand austauschbar ist.

Vorteilhafterweise ist dabei der eine Gleitpartner mit einer Titankarbid(TiC)-Schicht und der andere mit einer Titannitrid(TiN)-Schicht versehen. Die Herstellung der Hartstoffschichten auf den Gleitpartnern kann nach einem geeigneten, bekannten Herstellungsverfahren, z.B. nach dem CVD (Chemical vapor deposition)-Verfahren auf die hochglanzpolierte Oberfläche erfolgen. Die dabei aufgetragenen Hartstoff-Schichten haben vorteilhafterweise Dicken von 1 bis 5 μm.

Die genannten Hartstoffpaarungen haben im Experiment gute Resultate bezüglich Verschleissfestigkeit und bezüglich ihrer Haftfestigkeit auf dem Substrat ergeben; Das Substrat besteht vorzugsweise aus einem Metall, in dem Eisen, Kobalt oder Titan zumindest als Basiswerkstoff enthalten ist. Selbstverständlich kann das Substrat auch ein in der Implantat-Technik gebräuchlicher anderer Werkstoff, wie z.B. ein Kunststoff oder ein Reinmetall, beispielsweise Reintitan, sein.

Im folgenden wird die Erfindung anhand eines Ausführungsbeispiels näher erläutert.

Die Pfannenschale einer metallenen Hüftgelenkspfanne auf Co-Cr-Mo-Basis wird mit Hilfe der erwähnten Herstellungsverfahren mit einer 2 bis 3 μm dicken TiC-Schicht versehen, nachdem ihre Oberfläche zuvor — ebenfalls auf bekannte Weise — hochglanzpoliert (Rauhheitsklasse N 4 oder besser) worden ist.

In gleicher Weise wird ein Gelenkkopf des Femurteils einer Hüftgelenkprothese ebenfalls bis zur gleichen Güteklasse hochglanzpoliert und anschliessend eine wiederum 2 - 3 μm dicke TiN-Schicht aufgebracht.

Beide Teile ergeben eine Totalprothese mit guten Gleiteigenschaften und fest haftenden Hartstoffüberzügen auf den Gleitflächen.

## Patentansprüche

1. Gelenkimplantat für die Implantation in den lebenden Körper, bestehend aus zwei Gleitpartnern mit aufeinander gleitend bewegten Gleitflächen, die mit einer Hartstoffbeschichtung aus einem Metallkarbid, -nitrid, -carbonitrid, -borid oder -silizid versehen sind, wobei der weichere der beiden Hartstoffe auf den operativ leichter auswechselbaren Gleitpartner aufgebracht ist.

2. Gelenkimplantat nach Anspruch 1, dadurch gekennzeichnet, daß die Dicke die Hartstoffschichten 1 bis 5 μm beträgt.

3. Gelenkimplantat nach Anspruch 1 oder 2, dadurch gekennzeichnet, daß der eine Gleitpartner mit einer Titankarbid (TiC)-Schicht und der andere mit einer Titannitrid (TiN)-Schicht versehen ist.

4. Gelenkimplantat nach einem der Ansprüche 1 bis 3, dadurch gekennzeichnet, daß das zu beschichtende Substrat aus Metall besteht, das als Basis Eisen (Fe), Kobalt (Co) oder Titan (Ti) enthält.

## Revendications

1. Implant articulaire destiné à être implanté dans le corps vivant, constitué de deux pièces complémentaires en glissement présentant des surfaces de glissement glissant l'une sur l'autre qui sont pourvues d'un revêtement dur en carbure, nitrure, carbonitrure, borure ou siliciure de métal, la plus molle des deux matières dures étant appliquée sur la pièce en glissement qui peut le plus facilement être changée par une opération.

2. Implant articulaire selon la revendication 1, caractérisé en ce que l'épaisseur des couches de matière dure est comprise entre 1 et 5 μm.

3. Implant articulaire selon la revendication 1 ou 2, caractérisé en ce que l'une des pièces complémentaires en glissement est pourvue d'une couche de carbure de titane (TiC) et l'autre, d'une couche de nitrure de titane (Tin).

4. Implant articulaire selon une quelconque des revendications 1 à 3, caractérisé en ce que le support à recouvrir est en métal à base de fer (Fe), de cobalt (Co) ou de titane (Ti).

**Claims**

1. A joint implant for implantation in the living body and comprising two slidingly associated parts having sliding surfaces which move slidingly on one another and which have a hard facing of a metal carbide or metal nitride or metal carbonitride or metal boride or metal silicide, the softer of the two hard facings being applied to whichever of the slidingly associated parts can more readily be replaced by surgery.

2. A joint implant according to claim 1, characterised in that the thickness of the hard facings is from 1 to 5 µm.

3. A joint implant according to claim 1 or 2, characterised in that one of the slidingly associated parts has a layer of titanium carbide (TiC) and the other has a layer of titanium nitride (TiN).

4. A joint implant according to any of claims 1—3, characterised in that the substrate to be coated is made of metal containing as base iron (Fe), cobalt (Co) or titanium (Ti).